Europäisches Patentamt

European Patent Office

Office européen des brevets

(11) Veröffentlichungsnummer: **0 145 953**
A2

# (12) EUROPÄISCHE PATENTANMELDUNG

(21) Anmeldenummer: 84113665.8

(22) Anmeldetag: 13.11.84

(51) Int. Cl.⁴: **G 01 K 5/62**, G 01 K 13/00

(30) Priorität: 26.11.83 DE 3342939

(43) Veröffentlichungstag der Anmeldung: 26.06.85
Patentblatt 85/26

(84) Benannte Vertragsstaaten: AT BE CH FR GB IT LI LU NL
SE

(71) Anmelder: Metz, Gerhard, Dipl.-Ing. (FH),
Benzstrasse 4/1, D-7441 Wolfschlugen (DE)

(72) Erfinder: Metz, Gerhard, Dipl.-Ing. (FH),
Benzstrasse 4/1, D-7441 Wolfschlugen (DE)

(74) Vertreter: Patentanwälte Phys. Bartels, Dipl.-Ing. Fink
Dipl.-Ing. Held, Lange Strasse 51,
D-7000 Stuttgart 1 (DE)

(54) Vorrichtung zum Anzeigen des Zustandes eines Eies.

(57) Eine Vorrichtung zum Anzeigen des Zustandes eines
Eies hat eine Temperaturmeßeinrichtung (10 bis 13, 15 und
16), deren Meßstelle (10) sich in einem Wärmespeicher (6)
befindet und mindestens auf einer Seite wärmeisoliert ist
und deren Anzeigeteil (15, 16) in Schwimmlage der Vorrichtung ablesbar ist.

Um eine einfach ausgebildete Vorrichtung zu schaffen,
welche sich höchstens geringfügig über den Wasserspiegel
erhebt, besteht der Wärmespeicher aus Schwermetall und
hat auf einer Oberseite eine Ausnehmung (7) zur Aufnahme
der Meßstelle. Die Wärmeisolierung ist aus einer Wärmeisolierschicht (14) gebildet, welche die die Meßstelle aufnehmende Ausnehmung auf ihrer Oberseite abdeckt (Zeichnung).

# Patentanwälte

European Patent Attorneys

**Phys. H.Bartels**
**Dipl.-Ing. H.Fink**
**Dr.-Ing. M.Held**

Zugelassene Vertreter
beim Europäischen Patentamt

Patentanwälte · Lange Straße 51 · D-7000 Stuttgart 1

17. November 1983 Sc
P 6883

Herr Dipl.-Ing. (FH) Gerhard Metz, Benzstraße 4/1, 7441 Wolfschlugen

"Vorrichtung zum Anzeigen des Zustandes eines Eies"

Die Erfindung bezieht sich auf eine Vorrichtung entsprechend dem Oberbegriff des Anspruches 1.

Bei einer bekannten Vorrichtung der vorgenannten Art ist die Temperaturmeßeinrichtung als längliches Kapillarthermometer ausgebildet, dessen verhältnismäßig lange Flüssigkeitssäule beim Gebrauch der Vorrichtung beträchtlich über den Spiegel der Flüssigkeit zum Kochen von Eiern übersteht und ein Abdecken des die Flüssigkeit enthaltenden Gefässes verhindert (DE-OS 26 55 854).

Der vorliegenden Erfindung liegt die Aufgabe zugrunde, eine einfach ausgebildete Vorrichtung zu schaffen, welche sich höchstens geringfügig über den Wasserspiegel erhebt. Diese Aufgabe wird durch die Merkmale im Kennzeichnungsteil des Anspruches 1 erfindungsgemäß gelöst. Die Vorrichtung kann etwa in der Größe eines Eies ausgebildet, zusammen mit Eiern in einfacher Weise gelagert und in dem zum Kochen von Eiern benutzten Behälter in gleicher Weise wie die zu kochenden Eier untergebracht werden. Der Anzeigeteil der schwimmenden Vorrichtung kann von oben abgelesen werden.

Telefon (07 11) 29 63 10 u. 29 72 95
Telex 7 22 312 (patwo d)

Postscheck Stuttgart (BLZ 600 100 70) 448 42 - 704
Deutsche Bank Stuttgart (BLZ 600 700 70) 1167485

Telefonische Auskünfte und Aufträge sind
nur nach schriftlicher Bestätigung verbindlich.

Die Merkmale des Anspruches 2 sind auf eine günstige Ausbildung der Vorrichtung gerichtet.

Die Merkmale des Anspruches 3 tragen zu einer geringen Bauhöhe der Vorrichtung bei.

Die Einhaltung einer Lage der Vorrichtung, in der die Anzeigeeinrichtung von oben ablesbar ist, auch auf einer ebenen Fläche wird durch die Merkmale des Anspruches 4 erreicht.

Die Merkmale des Anspruches 7 führen zu einer sicheren Anzeige der Anzeigeeinrichtung.

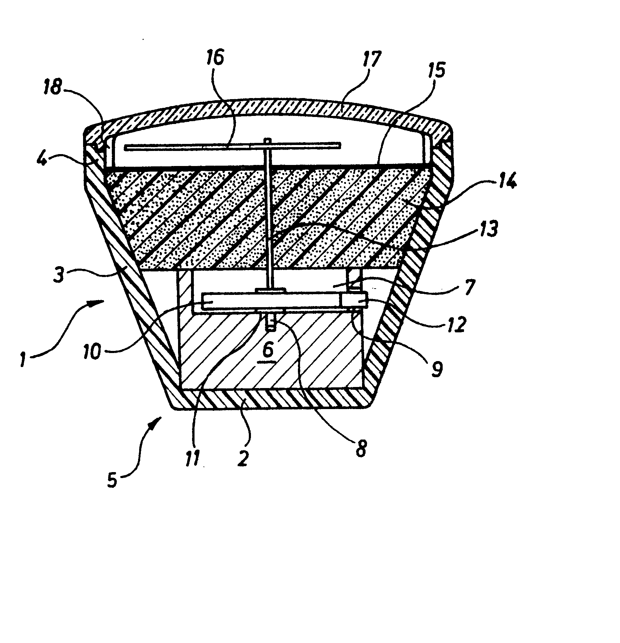

Weitere Vorteile ergeben sich aus den übrigen Ansprüchen, der Beschreibung und der Zeichnung. In dieser ist eine Vorrichtung zum Ablesen des Zustandes eines Eies als Ausführungsbeispiel des Gegenstandes der Erfindung im Längsschnitt schematisch dargestellt.

Ein Gehäuse 1 aus spritzbarem Kunststoff hat einen Boden 2, einen kegelstumpfförmigen Kegelteil 3 und auf seiner den größten Durchmesser aufweisenden Seite einen Zylinderteil 4. An dem Übergang vom Boden 2 zum Kegelteil 3 ist auf der Außenseite eine Wölbung 5 vorgesehen.

In das Gehäuse 1 ist ein Wärmespeicher 6 aus Schwermetall, insbesondere Eisen, Blei oder dgl., eingesetzt. Der Wärmespeicher ist im wesentlichen zylindrisch ausgebildet und hat auf seiner Oberseite eine zylindrische Ausnehmung 7. Er sitzt unmittelbar auf dem Boden 2 auf, ist in diesen etwas eingelassen und an diesem durch Kleben, Schrauben oder dgl. befestigt. In der Mitte des Bodens der Ausnehmung 7 befindet sich eine Bohrung 8 und auf der in der Zeichnung rechten Seite der Ausnehmung ein Durchgangsloch 9. In der Ausnehmung 7 ist eine Bimetallspirale 10 untergebracht, deren mittlerer Teil mit einer Klemmnabe 11 drehfest verbunden ist und deren radial äußerer Teil 12 das Durchgangsloch durchsetzt. Die Klemmnabe 11 ist mit einer Welle 13 drehfest verbunden, welche in die Bohrung 8 hineinragt und mit ihrem vom Boden 2 des Gehäuses 1 abgewandten Teil eine Wärmeisolierschicht 14 durchsetzt, die insbesondere aus einem Polyurethanschaum besteht. Die Isolierschicht hat etwa die gleiche Höhe wie der Wärmespeicher 6.

- -

17. November 1983 Sc
P 6883

Auf der vom Wärmespeicher 6 abgewandten Seite der Wärmeisolierschicht 14 ist ein Skalenblatt 15 befestigt, das ebenfalls von der Welle 13 durchsetzt ist, welche das Skalenblatt überragt und auf deren Ende im Abstand vom Skalenblatt ein Zeiger 16 angeordnet ist. Das Gehäuse 1 ist durch einen leicht gewölbten Deckel 17 aus durchsichtigem Werkstoff abgedeckt. Dieser Deckel 17 ist an dem oberen Rand des Zylinderteils 4 dicht befestigt und in gleicher Weise wie dieser abgestuft. Eine nach innen ragende Nase 18 oder eine entsprechende Stufe des Zylinderdeckels 17 reicht in axialer Richtung als Absatz bis zum Skalenblatt 15 und hält die Wärmeisolierschicht 14 auf dem Wärmespeicher 6.

Die Vorrichtung ist in der Weise ausgebildet, daß sie etwa die Wärmekapazität eines Eies hat und ist dessen Größe angepaßt. Der Kegelteil 3 kann in einen üblichen Eiaufnahmebehälter eingesetzt werden, wie er sich beispielsweise in einem Kühlschrank befindet. Wegen der Anordnung des Wärmespeichers 6 auf dem Boden 2 des Gehäuses 1 und wegen der Wölbung 5 am äußeren Rand des Bodens 2 ist die Vorrichtung bestrebt, stets eine Lage einzunehmen, in welcher die Welle 13 lotrecht angeordnet ist.

Beim Kochen von Eiern in einer Flüssigkeit wird die Vorrichtung in die gleiche Flüssigkeit eingelegt wie die Eier. Die Vorrichtung schwimmt in der Weise, daß praktisch nur der Deckel 17 aus der Flüssigkeit herausragt, so daß der Zeiger 16 und das Skalenblatt 15 durch den Deckel 17 hindurch noch sichtbar sind. Das Skalenblatt 15 kann in der Weise markiert werden, daß der Kochzustand eines Eies an der Stellung des Zeigers 16 zum Skalenblatt 15 ablesbar ist.

**Patentanwälte**
European Patent Attorneys

Phys. H. Bartels
Dipl.-Ing. H. Fink
Dr.-Ing. M. Held

Zugelassene Vertreter
beim Europäischen Patentamt

4/7

Patentanwälte · Lange Straße 51 · D-7000 Stuttgart 1

17. November 1983 Sc
P 6883

Herr Dipl.-Ing. (FH) Gerhard Metz, Benzstraße 4/1, 7441 Wolfschlugen

A n s p r ü c h e

1. Vorrichtung zum Anzeigen des Zustandes eines Eies mit einer Temperaturmeßeinrichtung (10 bis 13, 15 und 16) deren Meßstelle (10) sich in einem Wärmespeicher (6) befindet und mindestens auf einer Seite wärmeisoliert ist und deren Anzeigeteil (15, 16) in Schwimmlage der Vorrichtung ablesbar ist, dadurch g e k e n n z e i c h n e t , daß der Wärmespeicher aus Schwermetall besteht und auf seiner Oberseite eine Ausnehmung (7) zur Aufnahme der Meßstelle hat und daß die Wärmeisolierung aus einer Wärmeisolierschicht (14) gebildet ist, welche die die Meßstelle aufnehmende Ausnehmung auf ihrer Oberseite abdeckt.

2. Vorrichtung nach Anspruch 1, dadurch g e k e n n z e i c h n e t , daß der Wärmespeicher (6) und die Wärmeisolierschicht (14) in einem kegelstumpfförmigen Gehäuse (1) untergebracht sind, das auf seiner Oberseite von einem durchsichtigen Deckel (17) flüssigkeitsdicht abgedeckt ist, durch den hierdurch eine von der Meßstelle (10) beeinflußte Anzeigeeinrichtung (15, 16) ablesbar ist.

3. Vorrichtung nach Anspruch 1 oder 2, dadurch g e k e n n z e i c h n e t , daß

Telefon (07 11) 29 63 10 u. 29 72 95     Postscheck Stuttgart (BLZ 600 100 70) 448 42 - 704     Telefonische Auskunfte und Aufträge sind
Telex 7 22 312 (patwo d)                         Deutsche Bank Stuttgart (BLZ 600 700 70) 1167485     nur nach schriftlicher Bestätigung verbindlich.

17. November 1983 Sc
P 6883

die Meßstelle (10) aus einer Bimetallspirale besteht, die über eine die Wärmeisolierschicht (14) durchdringende Welle (13) mit einem Zeiger (16) der Anzeigeeinrichtung (15, 16) verbunden ist, welcher über eine Skala bewegbar ist.

4. Vorrichtung nach einem der vorhergehenden Ansprüche, dadurch g e k e n n - z e i c h n e t , daß der Wärmespeicher (6) im Gehäuse (1) unten angeordnet ist und unmittelbar auf dessen Boden (2) aufsteht und daß der äußere Rand des Boden (2) in einer Wölbung (5) in den Kegelteil (3) des Gehäuses übergeht.

5. Vorrichtung nach einem der vorhergehenden Ansprüche, dadurch g e k e n n - z e i c h n e t , daß der Deckel (17) innen einen Absatz (18) hat, mit dem er die Wärmeisolierschicht (14) an dem Wärmespeicher (6) hält.

6. Vorrichtung nach Anspruch 6, dadurch g e k e n n z e i c h n e t , daß der Absatz (18) des Deckels auf einem Skalenblatt (15) aufsteht, das auf der Isolierschicht (14) befestigt ist.

7. Vorrichtung nachh einem der vorhergehenden Ansprüche, dadurch g e k e n n - z e i c h n e t , daß sie etwa die Wärmekapazität eines Eies hat.